(19) Europäisches Patentamt
European Patent Office
Office européen des brevets

(11) **EP 2 526 927 A1**

(12) **EUROPÄISCHE PATENTANMELDUNG**

(43) Veröffentlichungstag:
**28.11.2012 Patentblatt 2012/48**

(51) Int Cl.:
***A61K 9/00*** *(2006.01)*　　***A61K 31/519*** *(2006.01)*

(21) Anmeldenummer: **11167515.3**

(22) Anmeldetag: **25.05.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Justus-Liebig-Universität Gießen**
**35390 Giessen (DE)**

(72) Erfinder:
• **Beck-Broichsitter, Moritz**
**35039, Marburg (DE)**

• **Gessler, Tobias**
**35435, Wettenberg (DE)**
• **Schmehl, Thomas**
**35392, Gießen (DE)**

(74) Vertreter: **Stumpf, Peter**
**c/o TransMIT GmbH**
**Kerkrader Strasse 3**
**35394 Gießen (DE)**

(54) **Biokompatible Nano- und Mikropartikel beschichtet mit Stabilisatoren für die pulmonale Applikation**

(57) Die vorliegende Erfindung stellt Stabilisatoren zur Beschichtung von biokompatiblen Nano- und Mikropartikeln bereit, die die Aggregation der Partikel bei der Herstellung, der Lagerung sowie vor und während der Vernebelung verhindern und zur Herstellung eines Mittels für die pulmonale Applikation verwendet werden können.

**EP 2 526 927 A1**

## Beschreibung

Die vorliegende Erfindung stellt mit Stabilisatoren beschichtete biokompatible Nano-und Mikropartikel bereit, die für die pulmonale Applikation von Wirkstoffen geeignet sind. Die Beschichtung der biokompatiblen Nano- und Mikropartikel mit einem Stabilisator bewirkt eine verbesserte Stabilität und Integrität der Partikel bei Vernebelung.

## Beschreibung des allgemeinen Gebietes der Erfindung

Die vorliegende Erfindung betrifft die Gebiete Innere Medizin, Pharmakologie, Nanotechnologie und Medizintechnik.

## Stand der Technik

Als eine selektive Route der Verabreichung pulmonaler Wirkstoffe ist dem Fachmann die Inhalation bekannt, wodurch ungewünschte systemische Nebenwirkungen umgangen werden können. Die direkte Applikation des Wirkstoffs in die Lunge erleichtert die gezielte Behandlung respiratorischer Erkrankungen, wie es zum Beispiel für das Prostazyklin-Analogon Iloprost (Ventavis®) bei der Behandlung der pulmonalen Hypertonie gezeigt worden ist. Die relativ kurze Dauer des pharmakologischen Effekts nach pulmonaler Wirkstoffdeposition ist jedoch ein großer Nachteil der Inhalationstherapie, die eine häufige Wirkstoffverabreichung durch Inhalation erfordert.

Kolloidale Trägermaterialien wie z.B. biokompatible Nano- und Mikropartikel sind als geeignete pulmonale Wirkstoff-Trägersysteme bekannt. Mit dem direkten Transport von Therapeutika, die in biokompatiblen Nano- und Mikropartikeln enthalten sind, in die Lunge kann eine langanhaltende und kontrollierte Wirkstoff-Freigabe am gewünschten Wirkort erreicht werden, die eine Verlängerung der pharmakologischen Effekte bewirkt.

Die Wahl der Herstellungsmethode der biokompatiblen Nano- und Mikropartikel hängt wesentlich von den physikochemischen Parametern des Polymers oder Lipids ab, das benutzt wird, sowie vom Wirkstoff, der in den biokompatiblen Nano- und Mikropartikeln verpackt wird. Die Auswahl des Polymers oder Lipids wird von Kriterien wie Biokompatibilität und Biodegradierbarkeit bestimmt. Zudem müssen die biokompatiblen Nano- und Mikropartikel weitere Standards erfüllen, wie beispielsweise eine ausreichende Assoziation des therapeutischen Agens mit dem Trägermaterial sowie Stabilität gegenüber den Kräften, die bei der Vernebelung auftreten. Diese stringenten Anforderungen werden von nanopartikulären Wirkstofftransportsystemen, die aus biokompatiblen Polymeren oder Lipiden bestehen, weitestgehend erfüllt. Bei herkömmlichen Formulierungen kann es während des Vernebelungsprozesses aus Suspension jedoch zu einer irreversiblen Aggregation der biokompatiblen Nano- und Mikropartikel kommen, welche zu einem Verlust der Funktionalität dieser Wirkstoff-Formulierung führt.

Es ist dem Fachmann zwar bekannt, dass Stabilisatoren die physikochemischen und biologischen Eigenschaften der verwendeten Formulierungen von biokompatiblen Nano- und Mikropartikel modulieren können, es wurden bislang jedoch keine Formulierungen mit Stabilisatoren beschrieben, welche die Stabilität und Integrität von biokompatiblen Nano- und Mikropartikeln bei Vernebelung ihrer Suspensionen verbessern und damit die Dauer der kontrollierten Wirkstoff-Freisetzung verlängern können.

Zusammenfassend weist der Stand der Technik Nachteile bezüglich der Stabilität und Integrität vernebelter, inhalierbarer Formulierungen von biokompatiblen Nano-und Mikropartikeln auf.

## Aufgabe

Aufgabe der Erfindung ist es, Stabilisatoren für biokompatible Nano- und Mikropartikel bereitzustellen, sodass bei Vernebelung ihrer Suspension eine verbesserte Stabilität und Integrität der Partikel erreicht wird und diese für die pulmonale Applikation von Wirkstoffen am Menschen besser geeignet sind.

## Lösung der Aufgabe

Die Aufgabe wird erfindungsgemäß durch die Ansprüche gelöst, insbesondere durch die Bereitstellung von Stabilisatoren ausgewählt aus der Gruppe der nichtionischen Tenside, anionischen Tenside, kationischen Tenside, amphoteren Tenside, Phospholipide oder der Polymere.

Die Stabilisatoren haben den Vorteil, dass sie an einer Vielzahl von Nano- und Mikropartikeln verwendet werden können. Sie sind biokompatibel und verlangsamen die Wirkstofffreisetzung aus den Partikeln, sodass ein enthaltener Wirkstoff über einen längeren Zeitraum am Wirkort freigesetzt wird verglichen mit Partikeln ohne Stabilisator.

Erfindungsgemäß ist der Stabilisator ausgewählt aus der Gruppe der nichtionischen Tenside, anionischen Tenside, kationischen Tenside, amphoteren Tenside, Phospholipide oder der Polymere. Nichtionische Tenside sind beispielsweise, aber nicht erschöpfend, PEG-PLGA, Poloxamere, Tween, Span, Pluronic. Geeignete Phospholipide sind beispielsweise Dipalmitoylphosphatidylcholin (DPPC), Lecithin, Distearoylphosphatidylcholin (DSPC) oder Dimyristoylphosphatidylcholin (DMPC). Geeignete Polymere sind beispielsweise die natürlichen Polymere, die synthetischen Polymere oder die halbsynthetischen Polymere.

Zu den natürlichen Polymeren gehören beispielsweise Proteine (z.B. Albumin), Cellulosen und deren Ester und Ether, Amylose, Amylopektin, Chitin, Chitosan, Collagen, Gelatine, Glykogen, Polyaminosäuren (z.B. Polylysin), Stärke, Dextrane oder Heparine.

Zu den synthetischen Polymeren gehören beispielsweise Polyethylenglykol (PEG), Polyethylenimin (PEI), Polyvinylalkohol (PVA), Polyvinylacetat, Polyvinylbutyrat, Polyvinylpyrrolidon (PVP), Polyacrylat, Poloxamere und Diblock- oder Triblockcopolymere aus PEG und Polyester (PLGA, PCL, PLA) (z.B. PEG-PLGA). Halbsynthetische Polymere sind zum Beispiel modifizierte Stärken (z.B. HES).

In einer bevorzugten Ausführungsform enthalten die erfindungsgemäßen beschichteten biokompatiblen Nano- und Mikropartikel Polyvinylalkohol, nachfolgend kurz als PVA bezeichnet, als Stabilisator. PVA ist ein kristalliner, wasserlöslicher Kunststoff.

Der Stabilisator ist besonders geeignet, um die damit beschichteten Partikel vorteilhaft mit piezoelektrischen, Düsen-, Ultraschall-Aerosolgeneratoren, Soft-Mist-Inhalern, oder Metered Dose Inhalern zu vernebeln, d.h. die Applikation in die Lunge erfolgt durch Inhalation eines Aerosols mit Hilfe eines Verneblers.

Die Stabilisatoren werden verwendet zur Herstellung von beschichteten biokompatiblen Nano- und Mikropartikeln, beispielsweise mit der Emulsionsmethode mit anschließender Lösungsmittelverdampfung (Evaporation). Die gebildeten dünnen protektiven Stabilisator-Schichten, die aus Tensiden, Phospholipiden oder Polymeren wie beispielsweise Polyvinylalkohol (PVA) bestehen, auf den erfindungsgemäßen beschichteten biokompatiblen Nano- und Mikropartikeln verbessern die Stabilität der Partikel bei der Vernebelung. Die Suspension der erfindungsgemäßen beschichteten biokompatiblen Nano- und Mikropartikel wird in ein Aerosol verwandelt, das für die Deposition in den respiratorischen Teil der Lunge geeignet ist. Die Charakteristika der erfindungsgemäßen beschichteten biokompatiblen Nano- und Mikropartikel werden nicht durch die Vernebelung beeinflusst. Die verlängerte Wirkstoff-Freigabe, die mit diesem neuen pulmonalen Wirkstoff-Transportsystem für pulmonale Wirkstoffe erreicht wird, führt dank des Stabilisators zu einer Verminderung der Wirkstoffgabe-Frequenz gegenüber herkömmlich verwendeten pharmazeutischen Mitteln, was die Lebensqualität und Therapietreue der Patienten verbessert.

Beispielhaft werden die Stabilisatoren zur Stabilisierung von biokompatiblen Nano-und Mikropartikeln verwendet. Vorzugsweise bestehen diese aus einem wasserunlöslichen biokompatiblen Polymer oder wasserunlöslichen Lipid sowie aus einem Stabilisator und einem Wirkstoff, der für die pulmonale Applikation geeignet ist. Die erfindungsgemäßen beschichteten biokompatiblen Nano- und Mikropartikel sind bevorzugt bioabbaubar.

Das biokompatible Polymer ist beispielsweise ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer.

Der Polyester ist bevorzugt ein lineares Poly(laktid-co-glykolid)-Copolymer (PLGA-Copolymer).

Es gibt geeignete PLGA-Copolymere für die Herstellung von beschichteten biokompatiblen Nano- und Mikropartikeln, die für die kontrollierte Wirkstoff-Freisetzung ("controlled release") verwendet werden. Diese umfassen beispielsweise, aber nicht erschöpfend, Copolymere aus der Resomer®-Familie. In einer bevorzugten Ausführungsform der Erfindung enthalten die biokompatiblen Nano- und Mikropartikel eine der folgenden Resomer®-Substanzen Resomer® Condensate RG 50:50 $M_n$ 2300, Resomer® R202S, Resomer® R202H, Resomer® R203S, Resomer® R203H, Resomer® R207S, Resomer® RG502H, Resomer® RG503H, Resomer® RG504H, Resomer® RG502, Resomer® RG503, Resomer® RG504, Resomer® RG653H, Resomer® RG752H, Resomer® RG752S, Resomer® RG753S, Resomer® RG755S, Resomer® RG756S oder Resomer® RG858S. In einer besonders bevorzugten Ausführungsform enthalten die erfindungsgemäßen beschichteten biokompatiblen Nano- und Mikropartikel das PLGA-Copolymer Resomer® RG502H.

Das wasserunlösliche Lipid ist beispielsweise ausgewählt aus der Gruppe der Acylglyceride (Mono-, Di- oder Triglycerid). Zu den Acylglyceriden gehört beispielsweise Glycerolmonopalmitat.

Als Wirkstoffe werden mit Vorteil solche eingesetzt, die ausgewählt sind aus der Gruppe der Appetitzügler/Antiadiposita, Azidosetherapeutika, Analeptika/Antihypoxämika, Analagetika, Antirheumatika, Anthelminthika, Antiallergika, Antianämika, Antiarrhythmika, Antibiotika, Antiinfektiva, Antidementiva, Antidiabetika, Antidota, Antiemetika, Antivertiginosa, Antiepileptika, Antihämorrhagika, Hämostatika, Antihypertonika, Antihypoglykämika, Antihypotonika, Antikoagulantia, Antimykotika, Antiparasitika, Antiphlogistika, Antitussiva, Expektorantia, Arteriosklerosemittel, ß-Blocker, Kalzium-Kanal-Blocker, Hemmstoffe des Renin-Angiotensin-Aldosteron-Systems, Broncholytika, Antiasthmatika, Cholagoga, Gallenwegstherapeutika, Cholinergika, Kortikoide, Diagnostika und Mittel zur Diagnosevorbereitung, Diuretika, Durchblutungsfördernde Mittel, Entwöhnungsmittel, Enzyminhibitoren, Enzymaktivatoren oder -stimulatoren, Präparate bei Enzymmangel, Rezeptorantagonisten, Transportproteine, Fibrinolytika, Geriatrika, Gichtmittel, Grippemittel, Mittel gegen Erkältungskrankheiten, Gynäkologika, Hepatika, Hypnotika, Sedativa, Hypophysen- oder Hypothalamushormone, regulatorische, Peptide, Hormone, Peptid-Hemmstoffe, Immunmodulatoren, Kardiaka, Koronarmittel, Laxantia, Lipidsenker, Lokalanästhetika, Neuraltherapeutika, Magen-Darm-Mittel, Migränemittel, Mineralstoffpräparate, Muskelrelaxanzien, Narkosemittel, Neurotrope Mittel, Osteoporosemittel, Kalzium-/ Knochenstoffwechsel-Regulatoren, Parkinsonmittel, Psychopharmaka, Sinusitismittel, Roborantia, Schilddrüsentherapeutika, Sera, Immunglobuline, Impfstoffe, Antikörper, Sexualhormone und ihre Hemmstoffe, Spasmolytika, Anticholinergika, Thrombozytenaggregationshemmer, Tuberkulosemittel, Urologika, Venentherapeutika, Vitamine, Zytostatika, Antineoplatische Mittel, Homöopathika, Gefäßaktive Substanzen, Gentherapeutika (DNA/RNA-Derivate), Transkriptions-Inhibitoren, Virostatika, Nikotin, Mittel gegen erektile Dysfunktion, Stickstoffmonoxid und/oder Stickstoffmonoxid-liberierende Substanzen.

Auch solche Partikel, die beispielweise in diagnostischen bildgebenden Verfahren, aber auch in der Therapie, z.B. in der Chemo- und Strahlentherapie und der Hyperthermie, zum Einsatz kommen, sind Wirkstoffe im Sinne der vorliegenden Erfindung.

Bei den Diagnostika handelt es sich sowohl um *in vitro*- als auch um *in vivo*-Diagnostika. Ein erfindungsgemäß einzusetzendes Diagnostikum ist beispielsweise bildgebend und/oder radioaktiv und/oder ein Kontrastmittel.

Insbesondere ist die Verwendung der erfindungsgemäß beschichteten biokompatiblen Nano- und Mikropartikel zur Herstellung eines pharmazeutischen Mittels zur Prävention, Diagnose und/oder Behandlung von Erkrankungen des Alveolarraumes sowie zur Behandlung von Erkrankungen der Atemwege und die Verwendung der beschichteten biokompatiblen Nano- und Mikropartikel zur Herstellung eines pharmazeutischen Mittels zur Prävention, Diagnose und/ oder Behandlung der pulmonalen Hypertonie durch größere Stabilität von besonderem Vorteil.

So können die erfindungsgemäß beschichteten biokompatiblen Nano- und Mikropartikel zur Herstellung von Mitteln zur Behandlung oder Diagnose folgender Erkrankungen eingesetzt werden: Entzündliche (infektiöse, nicht-infektiöse) und hyperproliferative (neoplastische, nicht-neoplastische) Erkrankungen der Lunge und der Atemwege, wie Bronchitis, COPD, Asthma, Pneumonie, Tuberkulose, pulmonale Hypertonie, Lungentumoren, fibrosierende Lungenerkrankungen. Weiterhin Lungenmetastasen, Mukoviszidose, Sarkoidose, Aspergillose, Bronchiekatsien, ALI, IRDS, ARDS, Alveolarproteinose, Immunsuppression und Infektionsprophylaxe nach Lungentransplantation.

Auch der Einsatz bei Sepsis, Fettstoffwechselstörungen, Tumorerkrankungen, Leukämien, angeborenen Stoffwechselstörungen (z. B. Wachstumsstörungen, Speicherstörungen, Störungen des Eisenhaushalts), endokrinologischen Erkrankungen, beispielsweise der Hypophyse oder der Schilddrüse (Glandula thyreoidea), Diabetes, Adipositas, psychischen Erkrankungen (z.B. Schizophrenie, Depression, bipolar-affektive Störungen, posttraumatisches Stressyndrom, Angst-und Panikstörungen), ZNS-Erkrankungen (beispielsweise M. Parkinson, Multiple Skerose, Epilepsie), Infektionskrankheiten, rheumatischen Erkrankungen, allergischen und Autoimmunerkrankungen, erektilen Dysfunktionen, Erkrankungen des Herz-Kreislaufsystems (beispielsweise Arterielle Hypertonie, Koronare Herzerkrankung, Herzrhythmusstörungen, Herzinsuffizienz, Thrombosen und Embolien), Niereninsuffizienz, Anämien, Antikörpermangelsyndromen, angeborenen oder erworbenen Gerinnungsstörungen, Thrombozytenfunktionsstörungen oder Vitaminmangelsyndromen ist denkbar.

Charakterisierung der erfindungsgemäß beschichteten biokompatiblen Nano- und Mikropartikel

Die erfindungsgemäß beschichteten biokompatiblen Nano- und Mikropartikel haben einen mittleren geometrischen Durchmesser zwischen 10 nm bis 10 $\mu$m, so dass sie gut vernebelbar sind, und eine Stabilisator-Schichtdicke zwischen 1 bis 200 nm. Die Stabilisator-Schichtdicke ist dabei jedoch nicht größer als der mittlere geometrische Radius der unbeschichteten biokompatiblen Nano- und Mikropartikel. In einer bevorzugten Ausführungsform haben die beschichteten biokompatiblen Nano- und Mikropartikel einen mittleren geometrischen Durchmesser von 500 nm bis 5 $\mu$m, um eine länger dauernde Wirkstoff-Freisetzung zu ermöglichen, oder zwischen 50 nm bis 250 nm, um eine Aufnahme der Partikel in Makrophagen zu verhindern.

Die beschichteten biokompatiblen Nano- und Mikropartikel enthalten zwischen 0 bis 50 % (w/w) und in einer bevorzugten Ausführungsform zwischen 1 bis 20 % (w/w) eines Wirkstoffs, der für die pulmonale Applikation geeignet ist.

Die erfindungsgemäß beschichteten biokompatiblen Nano- und Mikropartikel sind vorteilhaft mit piezoelektrischen, Düsen-, Ultraschall-Aerosolgeneratoren, Soft-Mist-Inhalern, oder Metered Dose Inhalern vernebelbar, d.h. die Applikation in die Lunge erfolgt durch Inhalation eines Aerosols mit Hilfe eines Verneblers. Der Durchmesser der erfindungsgemäßen beschichteten biokompatiblen Nano- und Mikropartikel ist für die Verneblung mit den genannten Verneblern besonders vorteilhaft, um in die tiefe Lunge gelangen zu können. Eine weitere Möglichkeit der Applikation in die Lunge besteht in der Instillation, beispielsweise über einen Katheter, ein Bronchoskop oder einen Beatmungszugang (z.B. Tubus oder Trachealkanüle). Darüber hinaus können sie zur Herstellung eines pharmazeutischen Mittels zur Prävention, Diagnose und/oder Behandlung von Erkrankungen verwendet werden.

Beschichtung von biokompatiblen Nano- und Mikropartikeln

Die biokompatiblen Nano- und Mikropartikel werden beispielsweise mit Hilfe der Emulsionsmethode und anschließender Lösungsmittelverdampfung (Evaporationsmethode) hergestellt.

Beispielhaft bestehen die zu beschichtenden Nano- und Mikropartikel aus einem wasserunlöslichen biokompatiblen Polymer oder wasserunlöslichen Lipid und einem Wirkstoff für die pulmonale Applikation. Das Polymer ist beispielsweise ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer.

Das wasserunlösliche Lipid ist beispielsweise ausgewählt aus der Gruppe der Acylglyceride (Mono-, Di- oder Triglycerid). Der Wirkstoff der biokompatiblen Nano-und Mikropartikel ist ausgewählt aus der Gruppe Appetitzügler/ Antiadiposita, Azidosetherapeutika, Analeptika/Antihypoxämika, Analagetika, Antirheumatika, Anthelminthika, Antiallergika, Antianämika, Antiarrhythmika, Antibiotika, Antiinfektiva, Antidementiva, Antidiabetika, Antidota, Antiemetika, Antivertiginosa, Antiepileptika, Antihämorrhagika, Hämostatika, Antihypertonika, Antihypoglykämika, Antihypotonika, Antikoagulantia, Antiomykotika, Antiparasitika, Antiphlogistika, Antitussiva, Expektorantia, Arteriosklerosemittel, β-Blocker, Kalzium-Kanal-Blocker, Hemmstoffe des Renin-Angiotensin-Aldosteron-Systems, Broncholytika, Antiasthmatika, Cholagoga, Gallenwegstherapeutika, Cholinergika, Kortikoide, Diagnostika und Mittel zur Diagnosevorbereitung, Diuretika, Durchblutungsfördernde Mittel, Entwöhnungsmittel, Enzyminhibitoren, Enzymaktivatoren oder -stimulatoren, Präparate bei Enzymmangel, Rezeptorantagonisten, Transportproteine, Fibrinolytika, Geriatrika, Gichtmittel, Grippemittel, Mittel gegen Erkältungskrankheiten, Gynäkologika, Hepatika, Hypnotika, Sedativa, Hypophysen- oder Hypothalamushormone, regulatorische Peptide, Hormone, Peptid-Hemmstoffe, Immunmodulatoren, Kardiaka, Koronarmittel, Laxantia, Lipidsenker, Lokalanästhetika, Neuraltherapeutika, Magen-Darm-Mittel, Migränemittel, Mineralstoffpräparate, Muskelrelaxanzien, Narkosemittel, Neurotrope Mittel, Osteoporosemittel, Kalzium-/ Knochenstoffwechsel-Regulatoren, Parkinsonmittel, Psychopharmaka, Sinusitismittel, Roborantia, Schilddrüsentherapeutika, Sera, Immunglobuline, Impfstoffe, Antikörper, Sexualhormone und ihre Hemmstoffe, Spasmolytika, Anticholinergika, Thrombozytenaggregationshemmer, Tuberkulosemittel, Urologika, Venentherapeutika, Vitamine, Zytostatika, Antineoplatische Mittel, Homöopathika, Gefäßaktive Substanzen, Gentherapeutika (DNA/RNA-Derivate), Transkriptions-Inhibitoren, Virostatika, Nikotin, Mittel gegen erektile Dysfunktion, Stickstoffmonoxid und/oder Stickstoffmonoxid-liberierende Substanzen.

Auch solche Partikel, die beispielweise in diagnostischen bildgebenden Verfahren, aber auch in der Therapie, z.B. in der Chemo- und Strahlentherapie und der Hyperthermie, zum Einsatz kommen, sind Wirkstoffe im Sinne der vorliegenden Erfindung.

Alternativ werden beschichtete biokompatible Nano- und Mikropartikel auch mittels Nanopräzipitation, Aussalzen, Polymerisation hergestellt. Diese genannten Herstellungsmethoden sind dem Fachmann bekannt.

Die Beschichtung der biokompatiblen Nano- und Mikropartikel mit einem Stabilisator erfolgt alternativ durch nachträgliches, nicht-kovalentes Beschichten mittels Mischen der unbeschichteten Partikel mit dem Stabilisator oder durch Beschichtung der Partikel aus der Gasphase mittels chemischer Gasphasenabscheidung (chemical vapour deposition) bzw. durch Besprühen der Partikel oder durch kovalentes Ansynthetisieren des Stabilisators an die unbeschichteten Partikel oder mittels Co-Electrospraying. Diese Methoden sind dem Fachmann bekannt.

Werden die biokompatiblen Nano- und Mikropartikel durch nachträgliches, nicht-kovalentes Beschichten mittels Mischen der unbeschichteten Partikel mit dem Stabilisator über die Emulsionsmethode hergestellt, so wird zunächst das Polymer oder Lipid unter Zugabe eines Wirkstoffs in einem Lösungsmittel gelöst. Anschließend wird die dabei entstehende dispergierte organische Phase in eine konstante wässrige Phase, die einen Stabilisator enthält, überführt. Nach Vermischen der beiden Phasen und Beschallung mit Ultraschall wird anschließend die organische Phase, die das Lösungsmittel enthält, durch Verdampfung entfernt und die erfindungsgemäß beschichteten biokompatiblen Nano- und Mikropartikel in Suspension erhalten. Geeignete Lösungsmittel, in welchen das erfindungsgemäß verwendete Polymer oder Lipid zu mindestens 0,1 % (w/w) löslich ist, sind beispielsweise, aber nicht erschöpfend, Dichlormethan, Chloroform, Ethylacetat, Benzylalkohol, Methylethylketon, Propylencarbonat. In einer bevorzugten Ausführungsform wird Polyvinylalkohol (PVA) als Stabilisator verwendet.

In einer bevorzugten Ausführungsform werden für die Herstellung der erfindungsgemäß beschichteten biokompatiblen Nano- und Mikropartikel biokompatibles Polymer zwischen 1 bis 100 g/l und Stabilisator zwischen 0,1 bis 25 g/l verwendet. In einer besonders bevorzugten Ausführungsform beträgt für die Herstellung die biokompatible Polymer-Konzentration 50 g/l und die Stabilisator-Konzentration 10 g/l.

Verwendung der erfindungsgemäß beschichteten biokompatiblen Nano- und Mikropartikel

Die erfindungsgemäß beschichteten biokompatiblen Nano- und Mikropartikel werden zur Herstellung eines pharmazeutischen Mittels zur pulmonalen Applikation eingesetzt. Biokompatibilität meint dabei die Verträglichkeit für das Gewebe und die Zellen am Wirkort, z.B. der Lunge.

Die Stabilität der erfindungsgemäß beschichteten biokompatiblen Nano- und Mikropartikel beruht auf der Verhinderung der Aggregation der Partikel bei der Herstellung, der Lagerung sowie vor und während der Vernebelung.

Sämtliche aus den Ansprüchen, der Beschreibung und den Zeichnungen hervorgehenden Merkmale und Vorteile einschließlich der Verfahrensschritte können sowohl für sich als auch in den verschiedensten Kombinationen erfindungswesentlich sein.

**Ausführungsbeispiele**

Die folgenden Ausführungsbeispiele 1 und 2 beschreiben die Charakterisierung des Stabilisators an beispielhaft beschichteten biokompatiblen Nano- und Mikropartikeln. Die Verwendung des Stabilisators ist nicht auf diese Partikel beschränkt. In den Ausführungsbeispielen wird beispielhaft Polyvinylalkohol (PVA) als Stabilisator verwendet und als Wirkstoff Sildenafil eingesetzt. Die erfindungsgemäß beschichteten biokompatiblen Nano- und Mikropartikel werden im Folgenden in Kurzform auch als Partikel bezeichnet. Poly(D,L-laktid-*co*-glykolid)-Copolymer (PLGA) wird nachfolgend auch kurz als Polymer bezeichnet.

1. Ausführungsbeispiel 1

*Herstellung der erfindungsgemäß beschichteten biokompatiblen Nano- und Mikropartikel mittels Emulsion und anschließender Evaporation*

Das Verfahren zur Beschichtung von biokompatiblen Nano- und Mikropartikeln mit einem Stabilisator ist gekennzeichnet durch die Schritte

a) Lösen des biokompatiblen Polymers oder Lipids und des Wirkstoffs in einem Lösungsmittel unter Bildung einer organischen Phase,
b) Emulgieren der organischen Phase in einer wässrigen Phase enthaltend einen Stabilisator,
c) Vermischen der organischen Phase aus Schritt a) mit der wässrigen Phase aus Schritt b),
d) Entfernen des Lösungsmittels und Erhalten der Partikel in Suspension.

Die erfindungsgemäß beschichteten biokompatiblen Nano- und Mikropartikel werden beispielsweise mit der Emulsionsmethode mit anschließender Lösungsmittelverdampfung, welche dem Fachmann bekannt ist, bei Raumtemperatur synthetisiert. Zuerst wird dazu zwischen 1 bis 100 g/l Poly(D,L-laktid-co-glykolid)-Copolymer (PLGA), das kommerziell erhältlich ist und beispielsweise als Resomer® RG502H von Boehringer Ingelheim (Ingelheim, Deutschland) bezogen werden kann, mit oder ohne Zugabe eines Wirkstoffs zwischen 1% bis 20%, wie beispielsweise der Phosphodiesterase-5-Inhibitor Sildenafil, welcher als freie Base kommerziell zum Beispiel von AK Scientific (Mountain View, Kalifornien, USA) erhältlich ist, in einem nicht mit Wasser mischbaren Lösungsmittel, wie zum Beispiel Methylenchlorid, gelöst. Dann werden 2 ml der organischen Phase (dispergierte Phase) in 10 ml einer wässrigen Phase (konstante Phase) enthaltend zwischen 0,1 bis 15 g/l eines Oberflächen-Stabilisators, der beispielsweise Polyvinylalkohol (PVA) ist, überführt. PVA ist kommerziell beispielsweise als Mowiol 4-88® von Sigma-Aldrich (Steinheim, Deutschland) erhältlich. Nach Vermischen der beiden Phasen wird die Emulsion mit Ultraschall behandelt. Die organische Phase wird anschließend langsam mit einem Rotationsevaporator durch Lösungsmittelverdampfung entfernt. Die Partikel werden direkt nach der Herstellung verwendet.

2. Ausführungsbeispiel 2

*Charakterisierung der erfindungsgemäß beschichteten biokompatiblen Nano- und Mikropartikel*

Die gemäß Ausführungsbeispiel 1 hergestellten beschichteten biokompatiblen Nano-und Mikropartikel werden mit Hilfe der nachfolgend unter Ausführungsbeispiel 2, Punkte 2.1 bis 2.5, beschriebenen Methoden und Ergebnisse charakterisiert. Dazu werden diese entweder direkt nach der Herstellung oder nach Vernebelung mit einem Vernebler, beispielsweise dem Aeroneb® Professional von Aerogen (Dangan, Galway, Irland), nach Herstellerangaben verwendet.

*2.1 Durchmesser, Größenverteilung und Oberflächenladung der erfindungsgemäß beschichteten biokompatiblen Nano- und Mikropartikel*

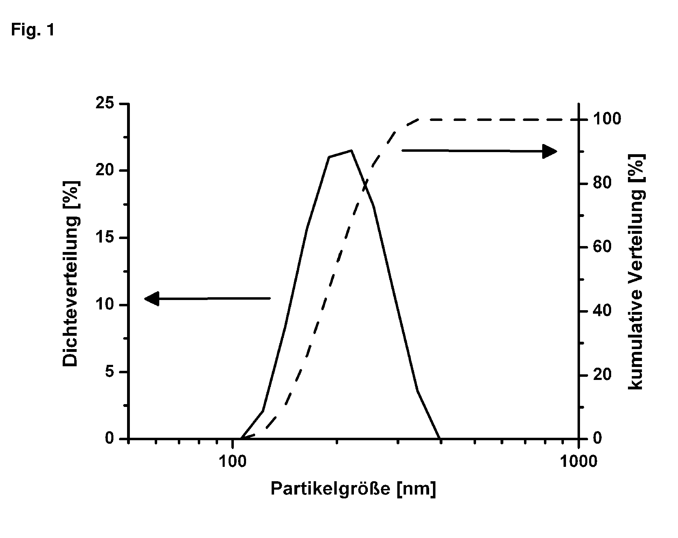

Frisch hergestellte beschichtete biokompatible Nano- und Mikropartikel, die mit Hilfe der Emulsionsmethode mit anschließender Lösungsmittelverdampfung wie unter Ausführungsbeispiel 1 beschrieben hergestellt werden, werden in verschiedenen Kombinationen der Polymer-Konzentration (zwischen 5 bis 100 g/l) und der PVA-Konzentration (zwischen 1 bis 50 g/l) auf ihre Eigenschaften Durchmesser, Größenverteilung und Oberflächenladung untersucht. Der hydrodynamische Durchmesser und die Größenverteilung (Polydispersität, PDI) der beschichteten biokompatiblen Nano- und Mikropartikel werden durch dynamische Lichtstreuung (DLS) gemessen. Das Zeta-Potenzial als Maß für die Oberflächenladung wird durch Laser-Doppler-Anemometrie (LDA), beispielsweise mit einem Zetasizer NanoZS/ZEN3600 (Malvern Instruments, Herrenberg, Deutschland), bestimmt. Alle Messungen werden bei einer Temperatur von 25°C mit Aliquots durchgeführt, die mit filtriertem und doppelt-destilliertem Wasser für die DLS bzw. 1,56 mM NaCl für die LDA verdünnt werden. Alle Messungen werden mindestens in Dreifachbestimmung mit mindestens 10 Durchläufen direkt nach der Präparation der beschichteten biokompatiblen Nano- und Mikropartikel durchgeführt. Dabei gibt n im Folgenden die Anzahl der Bestimmungen an.
Eine enge Partikelgrößenverteilung, d.h. Polydispersitätsindices (PDI), mit einem Wert kleiner 0,1 werden bei einer PVA-Konzentration größer 5g/l bei gleichbleibender PLGA-Konzentration von 50 g/l oder bei einer PLGA-Konzentration zwischen 10 bis 50 g/l bei gleichbleibender PVA-Konzentration von 10 g/l gefunden. Die Größenverteilung, die mit Hilfe von DLS bestimmt wird, frisch hergestellter beschichteter biokompatibler Nano- und Mikropartikel ist in Fig. 1 dargestellt. Die mittlere Größe der beschichteten biokompatiblen Nano- und Mikropartikel beträgt zwischen 100 und 400 nm (schwarze Linie in Abb. 1). Beschichtete biokompatible Nano- und Mikropartikel, die mit einer PLGA-Konzentration von 50g/l und einer PVA-Konzentration von 10 g/l hergestellt werden, weisen eine mittlere Partikelgröße von 195,1 $\pm$ 9,6 nm (Mittelwert $\pm$ Standardabweichung, n=4), eine enge Größenverteilung, d.h. einen engen Polydispersitätsindex (PDI), von 0,078 $\pm$ 0,002 (Mittelwert $\pm$ Standardabweichung, n=4) sowie eine negative Oberflächenladung, d.h. ein negatives Zeta-Potenzial, von -5,7 $\pm$ 0,8 mV (Mittelwert $\pm$ Standardabweichung, n=4) auf.

Um den Durchmesser, die Größenverteilung und die Oberflächenladung als Maß für die Stabilität der beschichteten biokompatiblen Nano- und Mikropartikel nach der Vernebelung zu untersuchen, werden die erfindungsgemäß beschichteten biokompatiblen Nano- und Mikropartikel mit einem theoretischen Gehalt von 5% (w/w) des Wirkstoffs Sildenafil (freie Base) gemäß Ausführungsbeispiel 1 hergestellt und vor und nach Vernebelung mit dem Vernebler Aeroneb® Professional charakterisiert. Dazu werden die vernebelten Suspensionen der beschichteten biokompatiblen Nano- und Mikropartikel gesammelt und qualitativ wie bei Dailey et al. beschrieben (Dailey LA, Kleemann E, Wittmar M et al.: Surfactant-free, biodegradable nanoparticles for aerosol therapy based on the branched polyesters, DEAPA-PVAL-g-PLGA. Pharm. Res. 20(12), 2011-2020 (2003); Dailey LA, Schmehl T, Gessler T et al.: Nebulization of biodegradable nanoparticles: impact of nebulizer technology and nanoparticle characteristics on aerosol features. J. Controlled Release. 86(1), 131-144 (2003)) untersucht. Die Suspensionen der beschichteten biokompatiblen Nano- und Mikropartikel werden bei einer Luftflußrate von 5 l/min vernebelt und durch Anbringen eines Glasobjektträgers direkt vor dem T-förmigen Mundstück des Verneblers, was das Absetzen von Aerosoltröpfchen auf dem Objektträger ermöglicht, gesammelt. Die daraus resultierende Kondensationsflüssigkeit wird zur weiteren Analyse gesammelt. Die Stabilität der vernebelten beschichteten biokompatiblen Nano- und Mikropartikel wird mit Hilfe von DLS wie oben beschrieben untersucht.

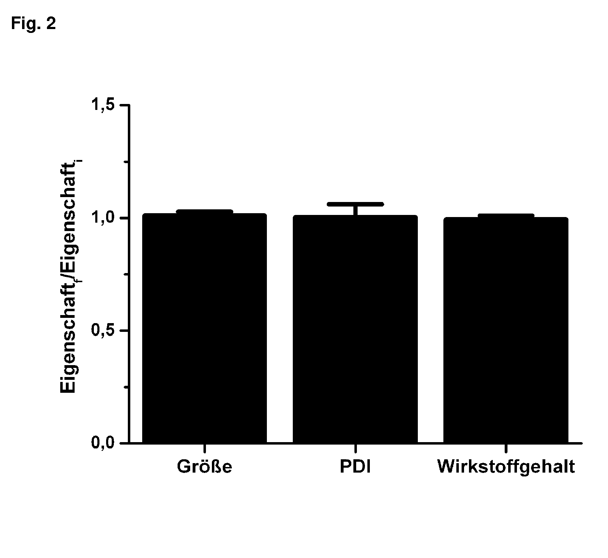

Die erfindungsgemäß beschichteten biokompatiblen Nano- und Mikropartikel weisen eine durchschnittliche Größe von 197,1 $\pm$ 1,7 nm, eine enge Größenverteilung mit einem PDI von 0,074 $\pm$ 0,005 sowie eine negative Oberflächenladung mit einem Zeta-Potenzial von -5,1 $\pm$ 0,3 mM auf. Die Parameter Partikelgröße, PDI und Sildenafil-Gehalt (siehe 2.3) werden in Fig. 2 als Quotient aus den Werten vor und nach Vernebelung dargestellt, Die Figur zeigt, dass die Vernebelung keinen signifikanten Einfluss auf die genannten Parameter hat.

*2.2 Schichtdicke des Stabilisators der erfindungsgemäß beschichteten biokompatiblen Nano- und Mikropartikel*

Die Dicke der adsorbierten PVA-Schichten, die als Oberflächenstabilisator der erfindungsgemäß beschichteten biokompatiblen Nano- und Mikropartikel fungieren, wird mit Hilfe der DLS- und der Zeta-Potenzial- Messungen wie unter Punkt 2.1 beschrieben als Funktion der Elektrolyt-Konzentration bestimmt. Geeignete Bestimmungsmethoden sind dem Fachmann bekannt. In Bezug auf die DLS-Messungen ist die adsorbierte PVA-Schichtdicke ($\delta$) abgeleitet vom Vergleich der Partikelgrößen von blanken ($d_0$) und beschichteten ($d_{ads}$) biokompatiblen Nano- und Mikropartikeln gemäß der folgenden Formel (1)

$$\delta = \frac{d_{ads} - d_0}{2} \qquad (1)$$

Die Schichtdicke aus den Zeta-Potenzial Messungen wird mit Hilfe der dem Fachmann bekannten Gouy-Chapman-Näherung kalkuliert, die die Abnahme des elektrostatischen Potenzials als eine Funktion der Distanz von der Oberfläche in der folgenden Formel (2) beschreibt.

$$\psi_x = \psi_0 \cdot e^{-\kappa x} \qquad (2)$$

Dabei ist $\psi_x$ das Potenzial bei einer Distanz x von der Oberfläche, $\psi_0$ ist das Oberflächenpotenzial und $K^{-1}$ ist die Debye-Länge. Eine Erhöhung der Elektrolyt-Konzentration (NaCl) erniedrigt die Debye-Länge. Zeta-Potenziale werden definiert als elektrostatische Potenziale an der Abscherebene, von denen angenommen wird, dass sie nur außerhalb der fixierten wässrigen Schicht eines biokompatiblen Nano-und Mikropartikels auftreten. Aus Gleichung (2) folgt Glei-

chung (3)

$$ln\,\psi_{x} = ln\,\psi_{0} - \kappa \cdot x \qquad (3)$$

Wenn Zeta-Potenziale ($\psi_x$) in verschiedenen Konzentrationen von NaCl (0, 0.1, 0.2, 0.5, 1, 2 und 5 mM) gemessen werden und gegen κ entsprechend $3.33 \cdot c^{1/2}$ aufgetragen werden, wobei c die Molalität von Elektrolyten ist, gleicht der Konzentrations-Anstieg die Dicke der adsorbierten Polymerschichten aus.

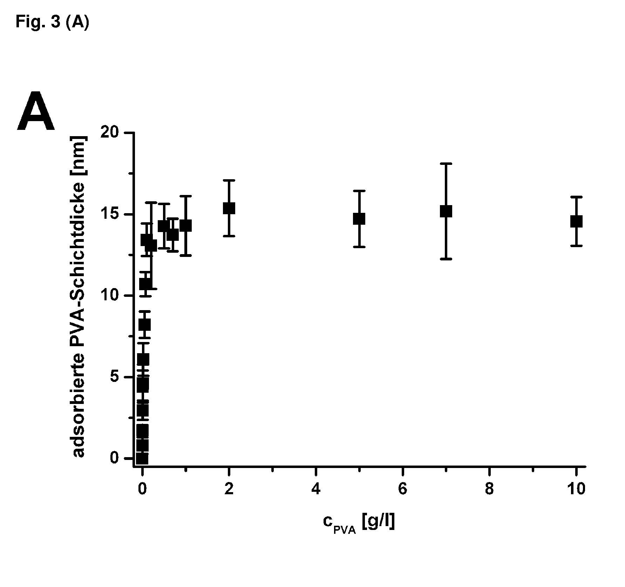

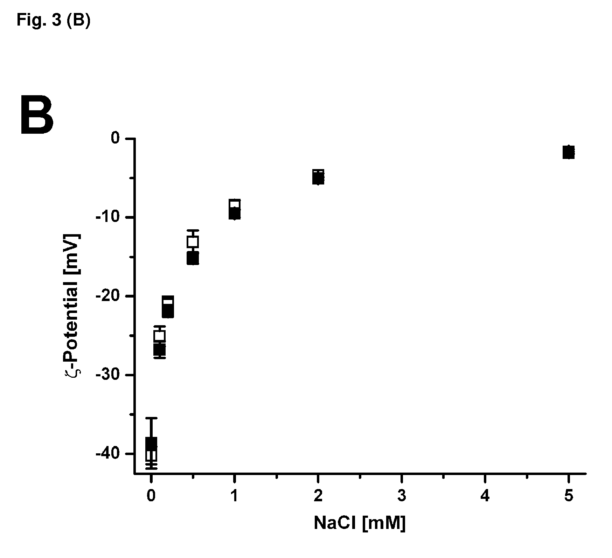

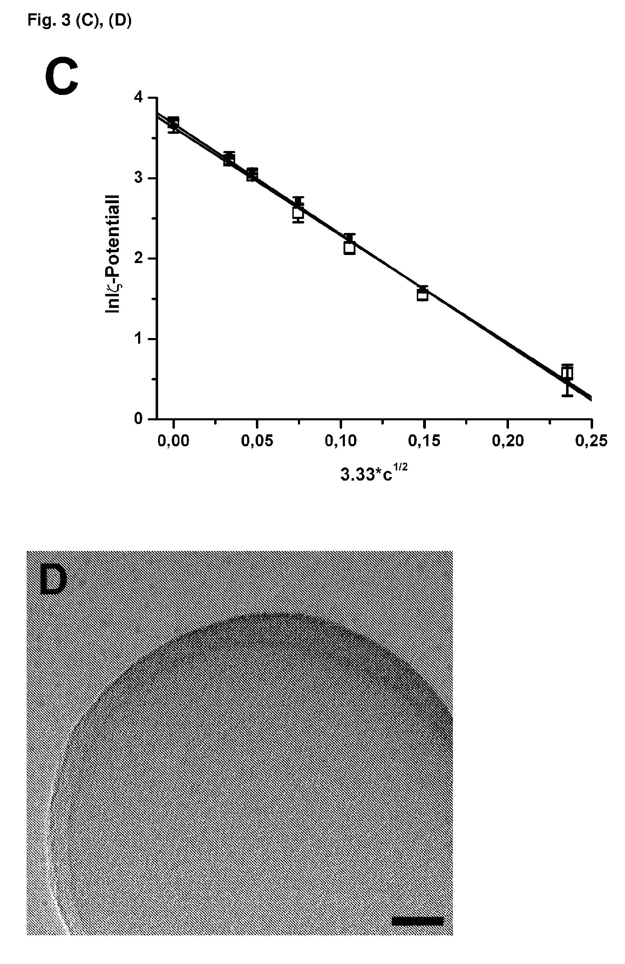

Fig. 3 stellt die adsorbierte PVA-Schichtdicke auf den beschichteten biokompatiblen Nano- und Mikropartikeln für frisch hergestellte (weiße Quadrate) als auch vernebelte Partikel (schwarze Quadrate) dar. In Fig. 3A ist diese in Abhängigkeit von der eingesetzten PVA-Konzentration gezeigt. Die Schichtdicke beträgt sowohl für frisch hergestellte als auch vernebelte Partikel zwischen 10 und 20 nm. Dieses Ergebnis wird auch durch transmissionselektronenmikroskopische Aufnahmen bestätigt. Dazu wird ein Kupfer-Grid (zum Beispiel S160-3, Plano, Wetzlar, Deutschland) mit einer dünnen Schicht einer verdünnten Suspension der beschichteten biokompatiblen Nano- und Mikropartikel beschichtet. Die beschichteten biokompatiblen Nano- und Mikropartikel werden dann auf dem Grid getrocknet und unter einem Transmissionselektronenmikroskop (TEM, beispielsweise JEM-3020 TEM, JEOL, Eching, Deutschland) bei einer Beschleunigung von 300 kV untersucht. Fig. 3D zeigt eine repräsentative TEM-Aufnahme eines erfindungsgemäß beschichteten biokompatiblen Nano- und Mikropartikels, bei der die PVA-Schicht (eingesetzte Konzentration bei der Herstellung gemäß Ausführungsbeispiel 1 gleich 10 g/l) deutlich zu erkennen ist. Das Zeta-Potenzial, also die Oberflächenladung, der Partikel ist bei allen getesteten NaCl-Konzentrationen negativ (Fig. 3B). Die gerade Linie in Fig. 3C zeigt den linearen Verlauf der experimentellen Daten.

*2.3 Stabilität und Integrität der erfindungsgemäß beschichteten biokompatiblen Nano- und Mikropartikel bei Vernebelung*

Um die Stabilität der erfindungsgemäß beschichteten biokompatiblen Nano- und Mikropartikel zu untersuchen, werden Aliquots von Suspensionen mit den Partikeln bei einer Luftflußrate von 5 l/min mit dem Aeroneb® Professional Vernebler gemäß Angaben des Herstellers vernebelt und die Aerosoltröpfchen als Kondensationsflüssigkeit auf einem Glasobjektträger aufgefangen. Die Kondensationsflüssigkeit wird anschließend mittels dynamischer Lichtstreuung (DLS) wie unter Punkt 2.1 beschrieben und Elektronenmikroskopie analysiert.

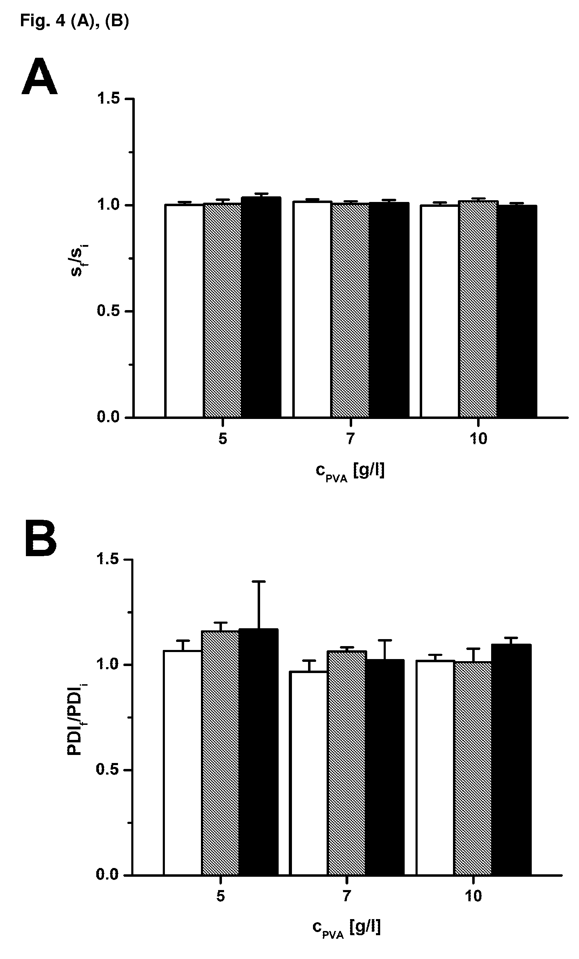

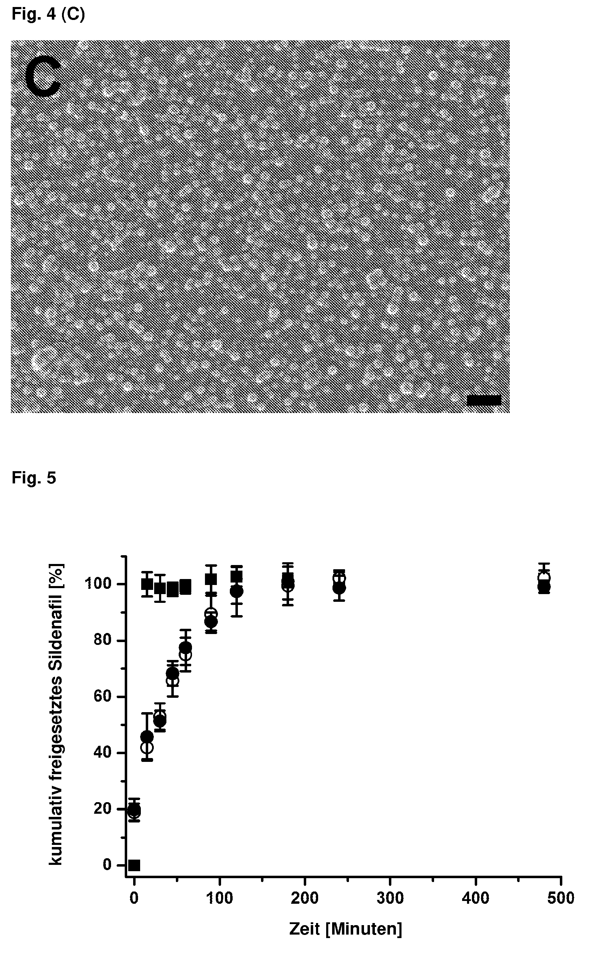

Fig. 4A und B zeigen, dass Partikelgröße und die Polydispersitätsindices durch die Vernebelung nicht beeinflusst werden. Auch die Integrität der Partikel bleibt bei der Vernebelung erhalten (Fig. 4C).

*2.4 Gehalt des Wirkstoffs in den erfindungsgemäß beschichteten biokompatiblen Nano- und Mikropartikeln*

Um den Gehalt an dem Wirkstoff Sildenafil der gemäß Ausführungsbeispiel 1 hergestellten beschichteten biokompatiblen Nano- und Mikropartikel zu bestimmen, wird beispielsweise 1 ml der Suspension der beschichteten biokompatiblen Nano-und Mikropartikel bei 16873 x g für 30 min bei 25°C zentrifugiert. Anschließend wird der Überstand vorsichtig abgenommen und darin die Menge von nicht eingeschlossenem Wirkstoff bestimmt. Die Pellets aus der Zentrifugation werden gefriergetrocknet, gewogen und anschließend beispielsweise in Chloroform, das als Lösungsmittel für PLGA und Sildenafil geeignet ist, gelöst. Die nicht gelöste Fraktion (Stabilisator) wird durch Zentrifugation abgetrennt. Anschließend wird aus der organischen Phase ein Aliquot entnommen, um die Menge an eingeschlossenem Sildenafil zu bestimmen. Die Konzentration von Sildenafil wird durch UV/Vis-Spektroskopie mit einem Spektrophotometer (beispielsweise Ultrospec® 3000, Pharmacia Biotech, Freiburg, Deutschland) bestimmt. Die Absorption aller Aliquots wird bei einer Wellenlänge von 291 nm gemessen. Der Gehalt an Wirkstoff (WST) in den beschichteten biokompatiblen Nano- und Mikropartikeln (PLGA-BNP) wird anhand einer Kalibrierungskurve kalkuliert und ist in der folgenden Formel (4) definiert.

$$WST\,Gehalt\ (\%\ (w/w)) = \frac{Masse\,WST\ in\ PLGA - BNP}{Masse\,PLGA - BNP} \cdot 100 \qquad (4)$$

Der Sildenafil-Gehalt ist zusammen mit den Parametern Partikelgröße und PDI (siehe 2.1) in Fig. 2 als Quotient aus den Werten vor und nach Vernebelung dargestellt, Die Figur zeigt, dass die Vernebelung keinen signifikanten Einfluss auf den Sildenafil-Gehalt hat.

*2.5 Freisetzung des Wirkstoffs aus den erfindungsgemäß beschichteten biokompatiblen Nano- und Mikropartikeln*

Untersuchungen zur Freisetzung des Wirkstoffs Sildenafil *in vitro* werden in phosphatgepufferter Salzlösung bei einem pH-Wert von beispielsweise 7,4 500 Minuten lang bei 37°C durchgeführt. Die Studien werden mit beschichteten biokompatiblen Nano- und Mikropartikeln durchgeführt, die einen theoretischen Wirkstoff-Gehalt von 5% (w/w) aufweisen. Aliquots der Suspensionen der beschichteten biokompatiblen Nano- und Mikropartikel werden in Glasröhrchen überführt und mit Medium, bestehend aus phosphatgepufferter Salzlösung (PBS) pH 7,4 + 0,1% Natriumdodecylsulfat (SDS), verdünnt. Die nachfolgende Inkubation erfolgt bei 37°C unter Schütteln der Aliquots. Parallel zu dem Versuchsansatz wird Wirkstoff Sildenafil allein in Medium unter den gleichen Bedingungen inkubiert. Eine Teilmenge wird zu vorgegebenen Zeitpunkten entnommen und zentrifugiert.

Die Freisetzung des Wirkstoffs Sildenafil aus den erfindungsgemäß beschichteten biokompatiblen Nano- und Mikropartikeln *in vitro* erfolgt über einen Zeitraum bis 300 Minuten (Fig. 5). Die Freisetzung aus Partikeln mit dem Polymer RG502H erfolgt über bis zu 90 Minuten. In diesem Zeitraum werden >95% Sildenafil kontinuierlich aus den Partikeln freigesetzt. Die Vernebelung mit dem Aeroneb® Professional hat dabei keinen Einfluss auf die Freisetzungsrate von Sildenafil.

**Abbildungslegenden**

Fig. 1

Größenverteilung der erfindungsgemäß beschichteten biokompatiblen Nano- und Mikropartikel, die durch dynamische Lichtstreuung (DLS) bestimmt wird. Die schwarze Linie repräsentiert die Dichte der Partikelgrößen, die gestrichelte Linie stellt die kumulative Verteilung der Partikelgrößen dar.

Fig. 2

Stabilität der erfindungsgemäß beschichteten biokompatiblen Nano- und Mikropartikel bei Vernebelung mit dem Vernebler Aeroneb® Professional. Die Stabilität ist gezeigt als Verhältnis der finalen zu den initialen Eigenschaften der erfindungsgemäßen Partikel (Eigenschaft$_f$/Eigenschaft$_i$) (A) (PDI = Polydispersitätsindex). Teilmengen der Suspensionen von beschichteten biokompatiblen Nano- und Mikropartikeln werden während der Vernebelung für die Analyse der Stabilität bei Vernebelung gesammelt. Die Werte sind als Durchschnittswerte $\pm$ Standardabweichung angegeben (n = 4).

Fig. 3

Adsorbierte Polyvinylalkohol (PVA) Schichtdicken auf den erfindungsgemäß beschichteten biokompatiblen Nano- und Mikropartikeln als Funktion der PVA-Konzentration ($C_{PVA}$) (A) und Zeta-Potenzial der beschichteten biokompatiblen Nano-und Mikropartikel, die in PVA-Lösung hergestellt wurden, als Funktion der Elektrolyt-Konzentration (B). Der Anstieg ($\kappa$) von In|Zeta-Potenzial| gegen 3.33*$c^{1/2}$ (Konzentration) ergibt die Dicke der adsorbierten Polymerschichten (C). Weiße und schwarze Quadrate in (B) und (C) repräsentieren die Charakteristika der frisch präparierten (B) bzw. vernebelten (C) biokompatiblen Nano- und Mikropartikel. Die gerade Linie in (C) repräsentiert den linearen Verlauf der experimentellen Daten ($R^2$ >0,99). Die adsorbierte PVA-Schicht ist in der repräsentativen transmissionselektronenmikroskopischen Aufnahme (D) sichtbar (Maßstab = 20 nm). Die Werte sind als Durchschnittswerte $\pm$ Standardabweichung angegeben (n = 4).

Fig. 4

Stabilität und Integrität von erfindungsgemäß beschichteten biokompatiblen Nano-und Mikropartikeln. Beispielhaft ist die Stabilität von PLGA-Nanopartikeln gezeigt, welche aus einer Lösung mit verschiedenen Konzentrationen des Stabilisators PVA ($C_{PVA}$) mit dem Aeroneb® Professional Vernebler nach Angaben des Herstellers vernebelt wurden. Die Stabilität ist angegeben als (A) Quotient der finalen (nach Vernebelung) zur initialen (vor Vernebelung) Partikelgröße ($s_f$/$s_i$) (Fig. 4A) und als (B) Quotient der Polydispersitätsindices (PDI) nach (PDI$_f$) und vor (PDI$_i$) Vernebelung (PDI$_f$/PDI$_i$) für verschiedene Nanopartikel-Konzentrationen (weiße Balken: 2 mg/ml; graue Balken: 5 mg/ml; schwarze Balken: 10 mg/ml) (Fig. 4B). Fig 4C zeigt eine repräsentative elektronenmikroskopische Aufnahme von Nanopartikeln nach Vernebelung (Maßstab = 1 $\mu$m). Die Werte sind als Durchschnittswerte $\pm$ Standardabweichung angegeben (n = 4).

Fig. 5

Profile der Freisetzung *in vitro* von Sildenafil aus den erfindungsgemäß beschichteten biokompatiblen Nano- und Mikropartikeln (Kreise). Weiße Kreise repräsentieren die Freisetzungseigenschaften frisch hergestellter beschichteter biokompatibler Nano- und Mikropartikel, während schwarze Kreise die Freisetzungseigenschaften vernebelter Partikel darstellen. Teilmengen von Suspensionen der beschichteten biokompatiblen Nano- und Mikropartikel werden während der Vernebelung für die Analyse des Einflusses der Verneblung auf das Freisetzungsprofil von Sildenafil aus den beschichteten biokompatiblen Nano- und Mikropartikeln gesammelt. Als Vergleich ist das Lösungsprofil der Pulversubstanz von Sildenafil (schwarze Quadrate) mit angegeben. Die Werte sind als Durchschnittswerte $\pm$ Standardabweichung angegeben (n = 4).

**Patentansprüche**

1. Verwendung von Stabilisatoren zur Beschichtung von biokompatiblen Nano- und Mikropartikeln zur Herstellung eines Mittels für die pulmonale Applikation, **dadurch gekennzeichnet, dass** sie ausgewählt sind aus der Gruppe der nichtionischen Tenside, anionischen Tenside, kationischen Tenside, amphoteren Tenside, Phospholipide oder Polymere, sodass die Aggregation der biokompatiblen Nano- und Mikropartikel verhindert wird.

2. Verwendung von Stabilisatoren gemäß Anspruch 1, **dadurch gekennzeichnet, dass** das Polymer Polyvinylalkohol (PVA) ist.

3. Verwendung der Stabilisatoren gemäß Anspruch 1 oder 2, **dadurch gekennzeichnet, dass** die Nano- und Mikropartikel aus biokompatiblem Polymer oder Lipid und einem Wirkstoff, der für die pulmonale Applikation geeignet ist, bestehen.

4. Verwendung gemäß Anspruch 3, **dadurch gekennzeichnet, dass** das biokompatible Polymer ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer ist.

5. Verwendung gemäß Anspruch 3, **dadurch gekennzeichnet, dass** das biokompatible Lipid ausgewählt ist aus der Gruppe der Acylglyceride.

6. Verwendung gemäß einem der vorangehenden Ansprüche, **dadurch gekennzeichnet, dass** der Stabilisator auf den biokompatiblen Nano- und Mikropartikeln eine Stabilisator-Schichtdicke zwischen 1 bis 200 nm aufweist.

7. Verfahren zur Beschichtung von biokompatiblen Nano- und Mikropartikeln mit einem Stabilisator gemäß einem der Ansprüche 1 bis 6, **gekennzeichnet durch** die Schritte

   e) Lösen des biokompatiblen Polymers oder Lipids und des Wirkstoffs in einem Lösungsmittel unter Bildung einer organischen Phase,
   f) Emulgieren der organischen Phase in einer wässrigen Phase enthaltend einen Stabilisator,
   g) Vermischen der organischen Phase aus Schritt a) mit der wässrigen Phase aus Schritt b),
   h) Entfernen des Lösungsmittels und Erhalten der Partikel in Suspension.

8. Verfahren zur Beschichtung von biokompatiblen Nano- und Mikropartikeln mit einem Stabilisator gemäß Anspruch 7, **dadurch gekennzeichnet, dass** das biokompatible Polymer ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer ist.

9. Verfahren zur Beschichtung von biokompatiblen Nano- und Mikropartikeln mit einem Stabilisator gemäß Anspruch 7, **dadurch gekennzeichnet, dass** das biokompatible Lipid ausgewählt ist aus der Gruppe der Acylglyceride.

Fig. 1

**Fig. 2**

**Fig. 3 (A)**

**Fig. 3 (B)**

Fig. 3 (C), (D)

**Fig. 4 (A), (B)**

**Fig. 4 (C)**

**Fig. 5**

Europäisches
Patentamt

European
Patent Office

Office européen
des brevets

# EUROPÄISCHER RECHERCHENBERICHT

Nummer der Anmeldung

EP 11 16 7515

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2010/009146 A1 (UNIV KANSAS [US]; BERKLAND CORY J [US]; BAILEY MARK [US]; EL GENDY NAS) 21. Januar 2010 (2010-01-21) * Beispiele 2,8,10 * ----- | 1-9 | INV. A61K9/00 A61K31/519 |
| X | EP 2 140 882 A1 (UNIV KYUSHU NAT UNIV CORP [JP]; KOWA CO [JP]) 6. Januar 2010 (2010-01-06) * Absatz [0016] - Absatz [0026] * * Beispiel 1 * ----- | 1-4,7,8 | |
| X | WO 2004/046202 A2 (TRANSMIT TECHNOLOGIETRANSFER [DE]; UNIV GIESSEN JUSTUS LIEBIG [DE]; UN) 3. Juni 2004 (2004-06-03) * Seite 7, Zeile 15 - Zeile 25 * * Beispiele 1-3 * * Ansprüche 1-23 * ----- | 1,3,4,6 | |
| A | CHRISTINE VAUTHIER ET AL: "Methods for the Preparation and Manufacture of Polymeric Nanoparticles", PHARMACEUTICAL RESEARCH, KLUWER ACADEMIC PUBLISHERS-PLENUM PUBLISHERS, NL, Bd. 26, Nr. 5, 24. Dezember 2008 (2008-12-24), Seiten 1025-1058, XP019686136, ISSN: 1573-904X * das ganze Dokument * ----- | 1-9 | RECHERCHIERTE SACHGEBIETE (IPC) A61K |

Der vorliegende Recherchenbericht wurde für alle Patentansprüche erstellt

| Recherchenort | Abschlußdatum der Recherche | Prüfer |
|---|---|---|
| München | 26. Januar 2012 | Sindel, Ulrike |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 16 7515

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-01-2012

| Im Recherchenbericht angeführtes Patentdokument | Datum der Veröffentlichung | Mitglied(er) der Patentfamilie | Datum der Veröffentlichung |
|---|---|---|---|
| WO 2010009146 A1 | 21-01-2010 | KEINE | |
| EP 2140882 A1 | 06-01-2010 | CA 2685054 A1<br>EP 2140882 A1<br>KR 20100015758 A<br>US 2010086615 A1<br>WO 2008139703 A1 | 20-11-2008<br>06-01-2010<br>12-02-2010<br>08-04-2010<br>20-11-2008 |
| WO 2004046202 A2 | 03-06-2004 | AU 2003294626 A1<br>CA 2545877 A1<br>DE 10253623 A1<br>EP 1562559 A2<br>US 2006127485 A1<br>WO 2004046202 A2 | 15-06-2004<br>03-06-2004<br>03-06-2004<br>17-08-2005<br>15-06-2006<br>03-06-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **DAILEY LA ; KLEEMANN E ; WITTMAR M et al.** Surfactant-free, biodegradable nanoparticles for aerosol therapy based on the branched polyesters. *DEAPA-PVAL-g-PLGA. Pharm. Res.,* 2003, vol. 20 (12), 2011-2020

- **DAILEY LA ; SCHMEHL T ; GESSLER T et al.** Nebulization of biodegradable nanoparticles: impact of nebulizer technology and nanoparticle characteristics on aerosol features. *J. Controlled Release,* 2003, vol. 86 (1), 131-144